Europäisches Patentamt

European Patent Office

Office européen des brevets

(11) Publication number: **0 323 632 B1**

(12) **EUROPEAN PATENT SPECIFICATION**

(45) Date of publication of patent specification: **15.07.92**  (51) Int. Cl.⁵: **A61K 6/06**, A61L 27/00,
                                                                A61K 6/08

(21) Application number: **88121787.1**

(22) Date of filing: **28.12.88**

The file contains technical information submitted
after the application was filed and not included in
this specification

(54) **Composition for forming calcium phosphate type setting material and process for producing setting material.**

(30) Priority: **28.12.87 JP 332904/87**
            **10.06.88 JP 143122/88**
            **22.06.88 JP 153730/88**

(43) Date of publication of application:
   **12.07.89 Bulletin  89/28**

(45) Publication of the grant of the patent:
   **15.07.92 Bulletin  92/29**

(84) Designated Contracting States:
   **DE GB NL**

(56) References cited:
   **EP-A- 0 147 021**
   **WO-A-86/01113**
   **US-A- 4 518 430**

   **JAPANESE PATENTS GAZETTE, section CH,
   week 8830, abstract no. 88-208078/30, 7th
   September 1988, Derwent Publications Ltd,
   London, GB**

   **PATENT ABSTRACTS OF JAPAN, vol. 11, no.
   192 (C-429)[2639], 19th June 1987**

(73) Proprietor: **ASAHI KOGAKU KOGYO
   KABUSHIKI KAISHA
   36-9, Maeno-cho 2-Chome Itabashi-ku
   Tokyo 174(JP)**

(72) Inventor: **Sumita, Masaya
   Asahi Kogaku Kogyo K.K. 36-9, Maeno-cho
   2-chome
   Itabashi-ku Tokyo(JP)**

(74) Representative: **Brauns, Hans-Adolf, Dr. rer.
   nat. et al
   Hoffmann, Eitle & Partner, Patentanwälte Ar-
   abellastrasse 4
   W-8000 München 81(DE)**

## Description

The present invention relates to a composition for forming calcium phosphate type setting material and a process for producing such a setting material. More particularly, the present invention relates to a composition for forming a calcium phosphate type setting material which is useful as a medical or dental cement material or a bone prosthesis. The present invention also relates to a process for producing a setting material in block form or other setting materials useful as artificial bones and dental roots.

Calcium phosphate compounds, in particular hydroxyapatite, have an excellent biocompatibility and their use as biomaterials in medical or dental fields has been widely investigated.

Calcium phosphate compounds other than apatite are known to be converted to hydroxyapatite upon hydrolysis under certain conditions (such as acidic conditions) and set at the same time. In an attempt to exploit this phenomenon, several efforts have been made to use calcium phosphate powders as dental or medical cement materials (as described, for example, JP-A-63-12705, 59-182263 and 59-88351) (the term "JP-A" as used herein means an unexamined published Japanese patent application)

Calcium phosphate powders are set by mixing them with a setting solution. In order to ensure that the setting material will do no harm to body tissues, it is desirable for the mixture to have a low pH and that the setting reaction will proceed in the neutral range. An aqueous solution of citric acid has heretofore been considered the best setting solution capable of satisfying these requirements. However, such an aqueous solution of citric acid still leaves room for improvement, especially when compared with acrylic cement materials which have been commonly used in the art. The considerable problem is that a mixture of a calcium phosphate powder and an aqueous citric acid solution is not gum-like and cannot be readily formed into a desired shape.

An object of the invention is to provide an improved composition for forming a calcium phosphate type setting material. This composition employs a powder component which can be produced by any suitable method; is highly gum-like; can be readily shaped into a desired form by kneading operations; allows a setting reaction to proceed uniformly and thoroughly in the neutral range; and produces a setting material which has high strength and yet does not harm body tissues.

Another object of the present invention is to provide a process for producing such a calcium phosphate type setting material.

Other objects and effects of the present invention will be apparent from the following description.

The present invention provides, in one aspect, a composition for forming a calcium phosphate type setting material comprising

(1) powder comprising at least one of α-tricalcium phosphate and tetracalcium phosphate; and

(2) a setting solution comprising an aqueous acidic solution having dissolved therein at least one polysaccharide selected from carboxymethyl chitin, glycol chitin, pullulan, high methoxy-pectin and chitosan in such an amount that the setting solution has a viscosity of 70 mPa.s (centi poise) or more.

## BRIEF DESCRIPTION OF THE DRAWING

Fig. 1 is a X-ray diffraction scan of the mixture of α-tricalcium phosphate and hydroxyapatite prepared in Example 1;

Fig. 2 is a X-ray diffraction scan of the calcium phosphate synthesized in Example 17; and

Fig. 3 is a X-ray diffraction scan of the powder prepared in Example 17 in accordance with the present inveniton.

As a result of various studies conducted in order to solve the aforementioned problems of the prior art, the present inventor has found that:

a composition for forming a calcium phosphate type setting material having good formability can be made from a powder component of calcium phosphate compound by using an aqueous acidic setting solution containing a polysaccharide in such an amount that the setting solution hag a viscosity at room temperature of 70 mPa.s (centi poise) or more, preferably from 100 to 20,000 mPa.s (cp,) and more preferably from 500 to 10,000 mPa.s (cp;)

a composition capable of being set mildly can be made by using an aqueous acidic setting solution containing at least one of a monosaccharide, an oligosaccharide, a sugaralcohol and a polyhydric alcohol;

setting materials of good quality can be produced from the above compositions; and

a uniform mixture of α-tricalcium phosphate and tetracalcium phosphate can be prepared by calcining, at a temperature of from 1,150 to 1,450°C under reduced pressure, a calcium phosphate having a molar ratio of Ca/P of about 1.8 or less and more than 1.5.

The present invention has been accomplished on the basis of these findings.

2

In the present invention, powders comprising at least one of $\alpha$-tricalcium phosphate and tetracalcium phosphate are used as a powder component of the composition. The powder component may further comprises at least one of $\beta$-calcium phosphate and hydroxyapatite. When these calcium phosphate powders are used as a mixture of two or more of them, the mixing ratio is not particularly limited, but the ratio of the total amount of $\alpha$-tricalcium phosphate and tetracalcoum phosphate to the total amount of the other components in the powder component is preferably 1/3 by weight or more in view of the activity on the setting reaction. These powder components need not be completely pure and they may contain minor amounts of impurities which are generated during the synthesis.

Among these powders, the following powders are preferably used in the present invention: (1) $\alpha$-tricalcium phosphate, (2) tetracalcium phosphate, (3) a mixture of $\alpha$-tricalcium phosphate and tetracalcium phosphate, (4) a mixture of $\alpha$-tricalcium phosphate and hydroxyapatite, (5) a mixture of tetracalcium phosphate and hydroxyapatite, (6) a mixture of tetracalcium phosphate and $\beta$-tricalcium phosphate, and (7) a mixture of $\alpha$-tricalcium phosphate and $\beta$-tricalcium phosphate.

The powder of $\alpha$-tricalcium phosphate (1) can be prepared by: the dry method in which calcium carbonate and calcium pyrophosphate (which is obtained by heating calcium hydrogenphosphate dihydrate at 550°C for 2 hours) are reacted each other at 1,200°C for 1 hour (as described, e.g., in Gypsum & Lime, No. 188, page 11 (1984) and Seitai Zairyo (Journal of Japanese Society for Biomaterials), vol. 4, No. 3, page 51 (1986)); or the wet method in which phosphoric acid and calcium hydroxide are reacted each other in an aqueous solution followed by calcining at from 1,120 to 1,180°C or higher temperatures (as described, e.g., in Bioceramics no Kaihatu to Rinsho (Bioceramics - Deveopment and Clinical Application), page 86, published by Quintessnce Shuppan, Japan on April 10, 1987).

The powder of tetracalcium phosphate (2) can be prepared by the dry method in which calcium carbonate and calcium pyrophosphate are reacted each other at 1,500°C (as described, e.g., in Shika Zairyo, Kikai (Journal of the Japanese Society for Dental Materials and Devices), vol. 5, special issue 7, page 50 (1986)).

The powder of a mixture of $\alpha$-tricalcium phosphate and tetracalcium phosphate (3) can be prepared by: the thermal decomposition method in which calcium phosphate having a molar Ca/P ratio of more than 1.5 and 1.8 or less is calculated at from 1,150 to 1,450°C under a reduced pressure, or in which hydroxyapatite obtained by the wet method is calcined at 1,500°C for 24 hours (as described in Transaction of Showa-62 Annual Meeting of Ceramics Society of Japan, page 931 (May 12, 1987); or mixing $\alpha$-tricalcium phosphate and tetracalcium phosphate each having been prepared separately (as described in Shika Zairyo, Kikai - (Journal of the Japanese Society for Dental Materials and Devices), vol. 5, special issue 7, page 50 (1986)).

The powder of a mixture of $\alpha$-tricalcium phosphate and hydroxyapatite (4) can be prepared by: the thermal decomposition method in which calcium phosphate having a molar Ca/P ratio of more than 1.5 and less than 1.67 is calcined at 1,000°C or more, preferably from 1,150 to 1,450°C; or mixing $\alpha$-tricalcium phosphate and hydroxyapatite each having been prepared seperetely (as described in JP-A-59-182263 and JP-A-59-88351).

The powder of hydroxyapatite can be prepared by: the dry method in which calcium carbonate and calcium pyrophosphate are reacted each other in steam (as described, e.g., in Bioceramics no Kaihatu to Rinsho (Bioceramics - Development and Clinical Application), page 53, published by Quintessence Shuppan, Japan on April 10, 1987); or the wet method in which phosphoric acid and calcium hydroxide are reacted each other in an aqueous solution (as described, e.g., in Bioceramics no Kaihatu to Rinsho - (Bioceramics - Deveopment and Clinical Application), page 53, published by Qintessence Shuppan, Japan on April 10, 1987).

The powder of a mixture of tetracalcium phosphate and hydroxyapatite (5) can be prepared by mixing tetracalcium phosphate and hydroxyapatite each having been prepared seperetely.

The pwder of a mixture of tetracalcium phosphate and $\beta$-tricalcium phosphate (6) can be prepared by mixing tetracalcium phosphate and $\beta$-tricalcium phosphate each having been prepared separately (as described in JP-A-59-182263).

The powder of $\beta$-tricalcium phosphate can be prepared by: the dry method in which calcium carbonate and calcium pyrophosphate are reacted each other at 1,000°C (as described, e.g., in J. Catal., vol. 75, page 200 (1982)); or the wet method in which phosphoric acid and calcium hydroxide are reacted each other in an aqueous solution followed by calcining at 800°C (as described, e.g., in Bioceramics no Kaihatu to Rinsho (Bioceramics - Deveopment and Clinical Application), page 86, published by Quintessnce Shuppan, Japan on April 10, 1987).

The method for preparing $\alpha$-tricalcium phosphate and $\beta$-tricalcium phosphate are different from each other in the calcining temperature. The formation of $\alpha$-tricalcium phosphate becomes predominant relative to the formation of $\beta$-tricalcium phosphate at a temperature range of from 1,120 to 1,180°C.

The powder of a mixture of α-tricalcium phosphate and β-tricalcium phosphate (7) can be prepared by: reacting phosphoric acid and calcium hydroxide followed by calcining at a temperature at which α-tricalcium phosphate and β-tricalcium phosphate are formed as a mixture; or mixing α-tricalcium phosphate and β-tricalcium phosphate each having been prepared separately.

The preparation of the powder of a mixture of α-tricalcium phosphate and tetracalcium phosphate (3) which is particularly preferably used in the present invention will be described in more detail below.

The two compounds, i.e., α-tricalcium phosphate and tetracalcium phosphate, may be synthesized separately and thereafter mixed in appropriate proportions.

Synthesis of α-tricalcium phosphate can be made by a known dry or wet process. Tetracalcium phosphate can be prepared by a known dry process involving the reaction between calcium pyrophosphate and calcium carbonate.

A mixture of α-tricalcium phosphate and tetracalcium phosphate can be prepared by the step of calcining, at a temperature of from 1,150°C to 1,450°C under reduced pressure, a calcium phosphate having a molar ratio of Ca/P of about 1.8 or less and more than 1.5 so as to produce a mixture of α-tricalcium phosphate and tetracalcium phosphate.

In this method for producing the mixture using a reduced pressure, the calcium phosphate having a molar ratio of Ca/P of more than 1.5 but not exceeding 1.8 can be readily synthesized by a wet method. If such calcium phosphate is calcined under reduced pressure at 1,150°C or more, it is thermally decomposed to form α-tricalcium phosphate and tetracalcium phosphate in admixture. The resulting mixture is uniform in composition. Such calcium phosphate generally starts to be thermally decomposed at a temperature near 1,400°C if the pressure is atmospheric. In the method described above, because the pressure is subatmospheric, the reaction of thermal decomposition is allowed to proceed at a fairly high speed even at the relatively low temperature of about 1,150°C. This is beneficial to the purpose of simplifying the manufacturing steps and reducing the production cost. The low calcining temperature offers the additional advantage of providing a highly active powder for use as a powder component for calcium phosphate type setting materials.

The calcium phosphate used as the starting material in the method described above can be readily synthesized by a wet process in which an aqueous solution of phosphoric acid is reacted with a suspension of calcium hydroxide by a known method. The molar ratio of Ca/P of this calcium phosphate must be more than 1.5 and 1.8 or less, preferably from 1.6 to 1.8. If the molar ratio of Ca/P is 1.5, the product thus-obtained does not become a mixture but α-tricalcium phosphate per se. If the molar ratio of Ca/P exceeds 1.8, calcium oxide which is deleterious to the human body will form during calcining. The Ca/P molar ratio of the calcium phosphate used as the starting material can be adjusted by changing the proportions of calcium hydroxide and phosphoric acid which are to be reacted in the synthesis process of the calcium phosphate. By changing the Ca/P molar ratio of the starting calcium phosphate, the proportions of α-tricalcium phosphate and tetracalcium phosphate to be finally produced can be adjusted to desired values.

After synthesis by a suitable method such as a wet process, the starting calcium phosphate is preferably rendered in powder form by suitable means such as filtration, centrifugation or spray-drying. It is also preferred for the calcium phosphate to be thoroughly dried to remove as much water as possibly by a suitable method such as precalcination at a temperature of from 500 to 700°C before it is thermally decomposed in a subsequent calcining step.

The thus prepared calcium phosphate is calcined under reduced pressure of 10 Pa or less at a temperature of from about 1,150°C to 1,450°C. If the calcining temperature is less than 1,150°C, the intended thermal decomposition reaction will not take place to a practically acceptable extent even if the pressure is decreased.

The reason why the calcium phosphate is calcined under reduced pressure is that the calcinating temperature can be lowered so as to simplify the manufacturing steps and to reduce the production costs, and that a product having a high activity can be attained. The reduced pressure under which the calcium phosphate is calcined is preferably about $10^{-2}$ Pa or less.

When the mixture of α-tricalcium phosphate and tetracalcium phosphate is prepared by calcining, at a temperature of from 1,150°C to 1,450°C under said reduced pressure, a calcium phosphate having a molar ratio of Ca/P of 1.8 or less and more than 1.5, because of the uniformity of the mixture, the activity on setting reaction of the mixture can be heightened and the setting reaction can proceed uniformly and sufficiently by which a setting material having a high strength can be obtained.

The preparation of the powder of a mixture of α-tricalcium phosphate and hydroxyapatite (4) which is particularly preferably used in the present invention is described in more detail below.

The two components, i.e., α-tricalcium phosphate and hydroxyapatite, may either be synthesized separately and thereafter mixed in appropriate propotions, or be produced simultaneously by calcining a

4

specific calcium phosphate, i.e., the thermal decomposition method. In the present invention, it is preferred that these two components are produced simultaneously by thermal decomposition.

In the case where the two components are produced separately, each of them may be prepared by any known method such as a wet method and a dry method, and then mixed in appropriate propotions by any known method such as by using a mortar or a ball mill.

In the case where the two components are produced simultaneously, a mixture of $\alpha$-tricalcium phosphate and hydroxyapatite used in the present invention is prepared by the step of calcining, at a temperature of 1,000°C or more, a calcium phosphate having a molar ratio of Ca/P of less than 1.67 and more than 1.5, preferably from 1.62 to 1.54.

The calcium phosphate used as a starting material for producing a mixture of $\alpha$-tricalcium phosphate and hydroxyapatite can be prepared by reacting a phosphoric compound with a calcium compound in which the amount of the phosphoric compound is in excess of the stoichiometric amount required for produce hydroxyapatite. For example, in a known method for producing hydroxyapatite by reacting an aqueous solution of phosphoric acid and a dispersion of calcium hydroxide, an excess amount of phosphoric acid is used to obtain the objective calcium phosphate.

The molar ratio of Ca/P of the calcium phosphate must be less than 1.67 and more than 1.5 and preferably from 1.62 to 1.54. If it is 1.5, $\alpha$-tricalcium phosphate per se, which is a component of the objective mixture, is produced. If it is 1.67, hydroxyapatite per se, which is a component of the objective mixture, is produced.

The molar ratio of Ca/P of the calcium phosphate can be controlled by changing the mixing ratio of calcium hydroxyide and phosphoric acid. The ratio of $\alpha$-tricalcium phosphate and hydroxyapatite of the mixture can be controlled by changing the molar ratio of Ca/P of the calcium phosphate.

The calcium phosphate is preferably produced by synthesizing by a wet method and then powderized by filtering, centrifuging, spray-drying, etc.

The thus prepared calcium phosphate is calcined at a temperature of 1,000°C or more, preferably from 1,150°C to 1,450°C. If the calcining temperature is less than 1,000°C, the intended thermal decomposition reaction will not take place to a practically acceptable extent, or only $\beta$-tricalcium phosphate is fromed which has a relatively low activity on the setting reaction even if the decomposition reaction takes place. If the calcining temperature is top high, the activity on setting reaction of the mixture is reduced.

When the mixture of $\alpha$-tricalcium phosphate and hydroxyapatite is prepared by calcining the calcium phosphate having a Ca/P molar ratio of more than 1.5 and less than 1.67, because of the uniformity of the mixture, the activity on setting reaction of the mixture can be heightened and the setting reaction can proceed uniformly and sufficiently by which a setting material having a high strength can be obtained.

In any of the above-described methods for producing calcium phosphate compounds, the wet methods can also be conducted according, e.g., to in Ann. Chim. (Paris), vol. 7, 808 and 823 (1952); J. Res. Nat. Bur. Stand., vol. 72A, 773 (1986); and Archs. Oral Biol., vol. 23, 329 to 336 (1978); and the dry methods can be conducted according, e.g., to Arch. Intern. Physiol. Biochim., vol. 72, 337 (1964); Chem. Abstr., vol. 60, 15418a (1964); and Studii Cercetari Chim., vol. 13, 157 (1962).

The mixture of $\alpha$-tricalcium phosphate and hydroxyapatite prepared by the above thermal decomposition method and the mixture of $\alpha$-tricalcium phosphate and tetracalcium phosphate prepared by the above thermal decomposition method can be preferably used as a powder component of the composition of the present invention and in the process for producing a setting material. Additionally, these mixtures may be used as a powder for thermal spray as described for example, in Kinzoku Hyomen Gijuttsu Binran - (Handbook of Metal Surface Treating Technology), pp. 1,132 to 1,139 (Nikkan Kogyo Shinbunsha, Japan, 1963) and JP-A-63-65871. If desired, the powder of these mixtures may be granulated, thereby producing a medical and dental granulated bone prosthesis. The granulated prosthesis has the advantage that it can be placed at will in any areas of any shape that need prosthetic treatments. Other applications of these mixtures include use as a column packing similar to the case of the hydroxyapatite.

The aqueous acidic solution for use as a setting solution in the present invention may contain various inorganic and organic acids dissolved therein. Examples thereof include inorganic acids such as phosphoric acid, and organic acids such as acetic acid, lactic acid, citric acid, malic acid, malonic acid, succinic acid, glutaric acid, tartaric acid and polyacrylic acid. In the present invention, these acids are used in aqueous solution having acid concentrations which are preferably about 25 wt% or more, more preferably from about 25 to 55 wt%. If the acid concentration of the aqueous acidic solution is less than about 25 wt%, the setting material obtained by mixing it with the powder component may not exhibit the desired strength.

Preferred examples of the aqueous acidic solution include a citric acid solution having an acid concentration of 40 wt%, a malic acid solution having an acid concentration of 40 wt%, a phosphoric acid solution having an acid concentration of 40 wt%, and a polyacrylic acid soilution having an acid concentration of 25 w%.

In accordance with one aspect of the present invention, a polysaccharide is added and dissolved in the various aqueous acidic solutions described above to form a setting solution. The polysaccharides preferably will not harm living tissues and have affinity for living tissues. Examples thereof include carboxymethyl chitin, glycol chitin, pullulan, high methoxy-pectin and chitosan with chitosan being particularly preferred. The term "chitosan" as used herein means a partially or completely deacetylated chitin. The deacetylation degree of chitosan and the substitution degree of carboxymethyl chitin and glycol chitin are not particularly limited.

The polysaccharides are dissolved in the aqueous acidic solutions in such an amount that the resulting setting solution has a viscosity at room temperature measured by a rotation viscometer of 70 mPa.s (cp) or more, preferably from 100 to 20,000 mPa.s (cp,) and more preferably from 500 to 10,000 mPa.s (cp.)

By using the polysaccharide-containing aqueous acidic solutions as setting solutions, the powder component can be set mildly in the neutral range, and the composition for forming setting materials is gum-like enough to be readily formed into any complex shape.

The setting reaction can be allowed to proceed even more mildly by further adding and dissolving at least one of a monosaccharide, an oligosaccaride, a sugaralcohol and a polyhydric alcohol in the polysaccharide-containing setting solution.

Examples of the monosaccharide include glucose and fructose, which may be used either alone or in admixture. Examples of the oligosaccharide include saccharose, maltose, lactose and raffinose, which may be used either alone or in admixture. Examples of the sugaralcohol include sorbitol, mannitol and xylitol, which may also be used either alone or in combination. Examples of the polyhydric alcohol include glycols (such as ethyleneglycol) and glycerine which may also be used either alone or in combination. Monosaccharides, oligasaccharides, sugaralcohols and polyhydric alcohol, which may be used either independently or in combination.

When at least one of monosaccharides, oligosaccharides, sugaralcohols and polyhydric alcohols are used, the setting reaction can proceed mildely and sufficiently even if the powder component having a high activity, e.g., a mixture of $\alpha$-tricalcium phosphate and tetracalcium phosphate, and a mixture of $\alpha$-tricalcium phosphate and hydroxyapatite which are prepared by the thermal decomposition, is used.

The total concentration of monosaccharides, oligosaccharides, sugaralcohols and polyhydric alcohols in the setting solution is preferably from about 5 to 40 wt%, more preferably from 10 to 30 wt%. If the concentration of these additives exceeds 40 wt%, increased difficulty will be encountered in dissolving them in the setting solution.

In any cases, the powder component and the setting solution are preferably mixed in such proportions that the ratio of the powder to the solution is within the range of from 0.4 to 2.7 by weight, more preferably from 0.4 to 2.0 by weight. If the ratio of the powder to the solution is less than 0.4, the solids content is too low to ensure desired strength for the setting material. If the ratio of the powder to the solution exceeds about 2.7, it becomes difficult to attain uniform mixing of the powder and the solution.

The setting material thus obtained by using the composition according to the present invention may further be calcined to improve the mechanical strength.

If a foaming agent (e.g., hydrogen peroxide) or a substance which disappears by calcining (e.g., polystyrene beads) is added to the material before completely setting, porous material can be obtained by calcining.

The following examples are provided for the purpose of further illustration the present invention. All parts, ratio and presents are by weight unless otherwise indicate.

## EXAMPLE 1

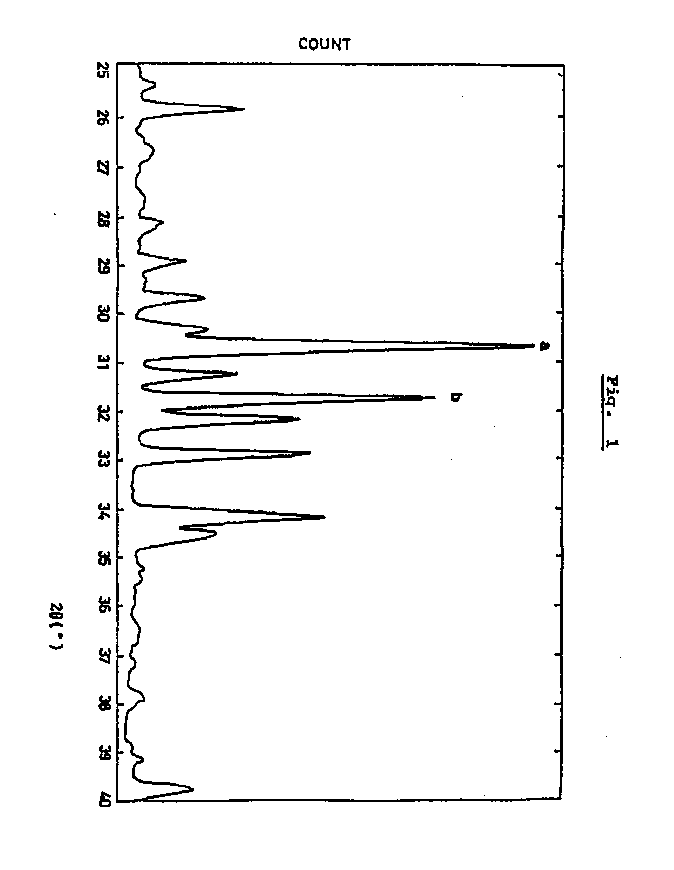

A calcium phosphate having a molar ratio of Ca/P of 1.57 was obtained by reacting an aqueous solution of phosphoric acid and a dispersion of calcium hydroxide in a conventional method followed by drying. The thus-obtained calcium phosphate was calcined at 1,200°C for 1 hour. The result of X-ray diffractiometry of the product obtained is shown in Fig. 1. In Fig. 1, peak a for $\alpha$-tricalcium phosphate and peak b for hydroxyapatite appeared which confirm that an mixture of $\alpha$-tricalcium phosphate and hydroxyapatite was formed by thermal decomposition of the calcium phosphate.

1 g of the mixture of $\alpha$-tricalcium phosphate and hydroxyapatite obtained above was mixed with 1 g of a setting solution prepared by dissolving 3 g of saccharose in 10 g of a 40% aqueous solution of citric acid to prepare a setting composition. The composition was set in about 10 minutes to produce a setting material having a high strength.

EXAMPLE 2

1 g of the mixture of α-tricalcium phosphate and hydroxyapatite obtained in Example 1 was mixed with 1 g of a setting solution composed of an aqueous solution of polyacrylic acid containing 1% of saccharose (acid concentration: about 25%, produced by Wako Pure Chemical Co., Ltd.) to prepare a setting composition. The composition was set in about 15 minutes to produce a setting material having a high strength.

EXAMPLE 3

1 g of the mixture of α-tricalcium phosphate and hydroxyapatite obtained in Example 1 was mixed with 1 g of a setting solution composed of a 40% aqueous solution of phosphoric acid containing 10% of saccharose to prepare a setting composition. The composition was set in about 30 seconds to produce a setting material having a high strength.

COMPARATIVE EXAMPLE 1

1 g of the mixture of α-tricalcium phosphate and hydroxyapatite obtained in Example 1 was mixed with 1 g of a setting solution composed of 40% aqueous solution of phosphoric acid. The mixture immediately reacted vigorously with a great amount of exothermic heat, and once produced a setting material, but the material was broken immediately.

EXAMPLES 4 TO 16

1 g of the mixture of α-tricalcium phosphate and hydroxyapatite obtained in Example 1 was mixed with 1 g of a setting solution shown in Table 1 below to prepare a setting composition.

## Table 1

| Example | Setting solution | | Setting time (min) |
|:---:|:---:|:---:|:---:|
| | Aqueous acidic solution | Additive | |
| 4 | 40% citric acid | 25% glucose | 10 |
| 5 | 40% citric acid | 25% sorbitol | 10 |
| 6 | 40% citric acid | 25% glycerin | 12 |
| 7 | 40% citric acid | 0.5% chitosan* | 1 |
| 8 | 40% malic acid | 25% glycerin | 14 |
| 9 | 40% malic acid | 25% saccharose | 15 |
| 10 | 40% phosphoric acid | 30% glycerin | 1 |
| 11 | 40% phosphoric acid | 0.5% chitosan* | 0.2 |
| 12 | 25% polyacrylic acid | 5% glucose | 15 |
| 13 | 25% polyacrylic acid | 5% sorbitol | 15 |

(continued)

8

### Table 1 (continued)

| Example | Setting solution | | Setting time (min) |
|---|---|---|---|
| | Aqueous acidic solution | Additive | |
| 14 | 40% citric acid | 25% saccharose + 0.5% chitosan* | 15 |
| 15 | 40% phosphoric acid | 25% saccharose + 0.5% chitosan* | 1 |
| 16 | 40% malic acid | 25% glycerin + 0.5% chitosan* | 17 |

Note:  * Chitosan was a commercial product, "Flonac N" made by Kyowa Yushi Kogyo K.K.

In Example 7, the viscosity at room temperature of the setting solution was 1,000 mPa.s (cp).

The composition was set in the period of time shown in Table 1 to produce a Betting material having a high strength. In the case where the setting solution contained chitosan as a polysaccharide, the mixture of the powder and the setting solution provided a plastic gum-like composition which was easily shaped into a desired form.

EXAMPLE 17

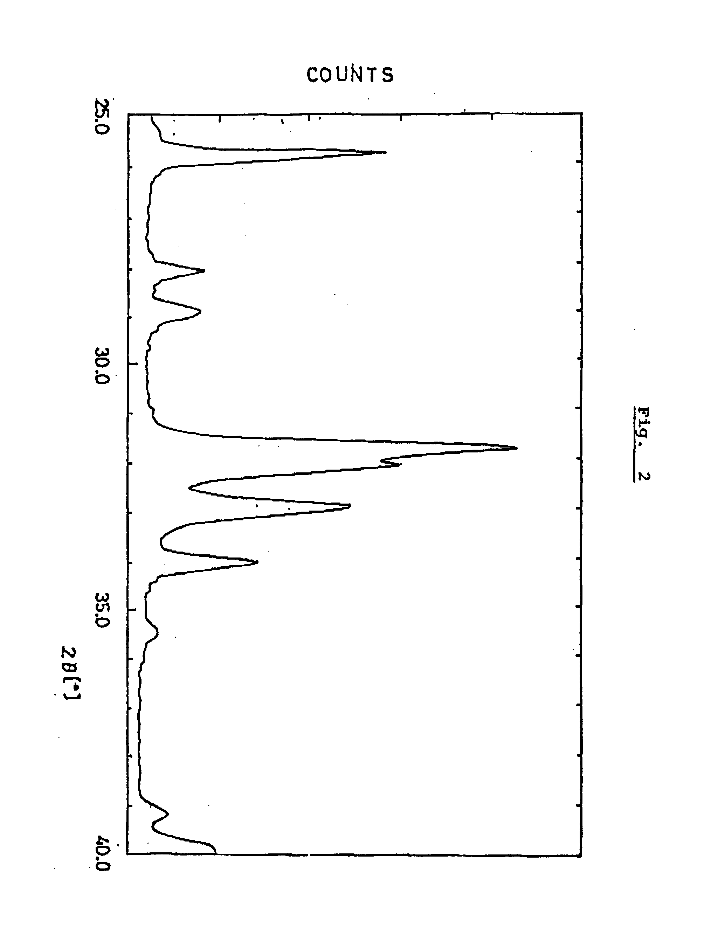

An aquous solution of phosphoric acid was reacted with a suspension of calcium hydroxide by a conventional method, and the reaction product was dried to obtain calcium phosphate. The identity of the product was established by X-ray diffraction, the results of which are shown by an X-ray diffraction scan in Fig. 2. A chemical analysis revealed that the resulting calcium phosphate had a Ca/P molar ratio of 1.67.

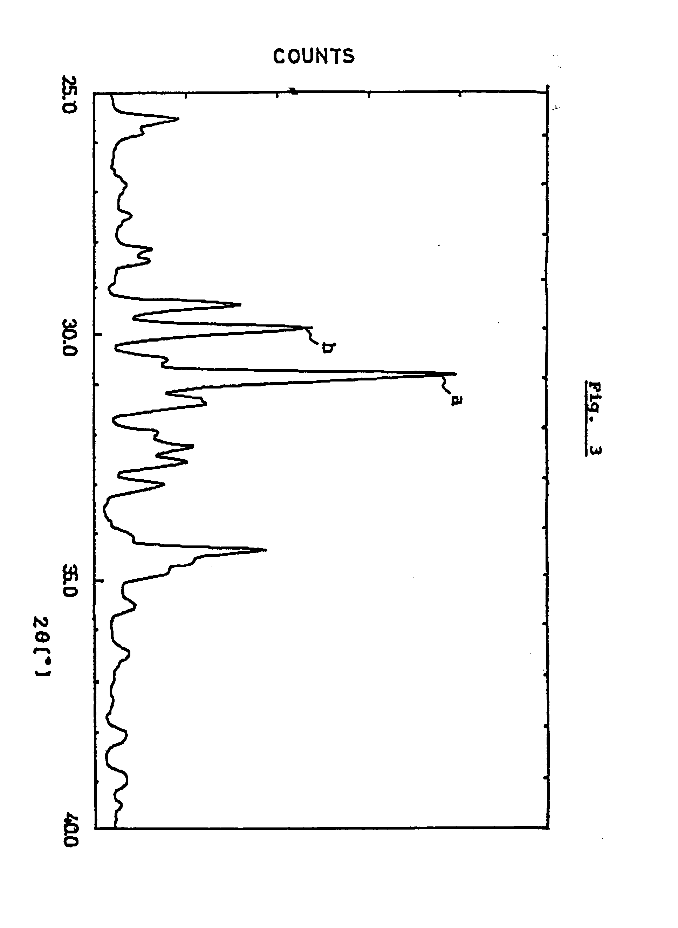

This calcium phosphate was calcined at 1,200°C for 1 hour at a pressure of $1.3 \times 10^{-4}$ Pa. The resulting product was analyzed by X-ray diffraction as above and the results are shown in Fig. 3. The appearance of two characteristic peaks a ($\alpha$-tricalcium phosphate) and b (tetracalcium phosphate) verified the production of a mixture of $\alpha$-tricalcium phosphate and tetracalcium phosphate by thermal decomposition of the calcium phosphate.

2 g of the above-obtained powder mixture of $\alpha$-tricalcium phosphate and tetracalcium phosphate was mixed with 1 g of a setting solution having a viscosity at room temperature of 200 mPa.s (cp) which was prepared by adding 3 g of glucose and 0.2 g of carboxymethyl chitin (CM-Chitin 100 made by Katokichi Co., Ltd.) to 10 g of a 45% aqueous solution of malic acid. The resulting plastic composition was highly gum-like and set in about 12 minutes to produce a setting material having a high strength.

EXAMPLE 18

The same procedures as in Example 17 were repeated except that 0.2 g of glycol chitin (made by Katokichi Co., Ltd.) was used instead of the carboxymethl chitin used in Example 17. The resulting plastic composition was highly gum-like and set in about 12 minutes to produce a setting material having a high strength.

EXAMPLE 19

The same procedures as in Example 17 were repeated except that 0.2 g of high methoxy-pectin (Rapid Set Baking 150SAG made by Mero Rousselot Satia) was used instead of the carboxymethl chitin used in Example 17. The viscosity at room temperature of the setting solution was 900 cp. The resulting plastic composition was highly gum-like and set in about 15 minutes to produce a setting material having a high strength.

EXAMPLE 20

The same procedures as in Example 17 were repeated except that 10 g of pullulan (PF20 made by Hayashibara Co., Ltd.) was used instead of the carboxymethl chitin used in Example 17. The resulting plastic composition was highly gum-like and set in about 20 minutes to produce a setting material having a high strength.

COMPARATIVE EXAMPLE 2

The same procedures as in Example 17 were repeated except that 0.2 g of methylcellulose (made by Wako Pure Chemical Co., Ltd.) was used instead of the carboxymethl chitin used in Example 17. The resulting composition was not gum-like but set in about 12 minutes to produce a setting material having a high strength.

EXAMPLE 21

As aqueous solution of phosphoric acid was reacted with a suspension of calcium hydroxide by a conventional method. The reaction product was dried and calcined at 1,200°C to obtain $\alpha$-tricalcium phosphate in powder form. 2 grams of this powder was mixed with 1 g of a setting solution having a viscosity at room temperature of 6,000 cp prepared by dissolving 0.1 g of chitosan ("Flonac N" made by Kyowa Yushi Kogyo K.K.) in 10 g of an aqueous solution of 40% citric acid. A plastic gum-like composition was obtained and this composition set in about 7 minutes to produce a setting material having a high strength.

EXAMPLE 22

An aqueous solution of phosphoric acid was reacted with a suspension of calcium hydroxide by a conventional method. The reaction product was dried and calcined at 1,200°C to obtain $\alpha$-tricalcium phosphate in powder form. 2 grams of this powder was mixed with 1 g of a setting solution prepared by dissolving 0.1 g of chitosan ("Flonac N" made by Kyowa Yushi Kogyo K.K.) and 3 g of saccharose in 10 g of an aqueous solution of 40% citric acid. A plastic gum-like composition was obtained and this composition set in about 10 minutes to produce a setting material having a high strength.

EXAMPLE 23

Calcium pyrophosphate was reacted with calcium carbonate by a conventional method, and calcined to obtain tetracalcium phosphate in powder form. 2 grams of this powder was mixed with 1 g of a setting solution prepared by dissolving 0.1 g of chitosan ("Flonac N" made by Kyowa Yushi Kogyo K.K.) and 3 g of saccharose in 10 g of an aqueous solution of 40% citric acid. A plastic gum-like composition was obtained and this composition set in about 5 minutes to produce a setting material having a high strength.

EXAMPLE 24

$\alpha$-Tricalcium phosphate and tetracalcium phosphate were prepared separately by conventional methods and mixed at a weight ratio of 2/1 to make a mixed powder. 2 grams of this mixed powder was mixed with 1 g of a setting solution prepared by dissolving 0.1 g of chitosan ("Flonac N" made by Kyowa Yushi Kogyo K.K.) in 10 g of an aqueous solution of 40% citric acid. A plastic gum-like composition was obtained and this composition set in about 5 minutes to produce a setting material having a high strength.

EXAMPLE 25

2 grams of the mixed powder prepared in Example 24 was mixed with 1 g of a setting solution prepared by dissolving 0.1 g of chitosan ("Flonac N" made by Kyowa Yushi Kogyo K.K.) and 3 g of saccharose in 10 g of an aqueous solution of 40% citric acid. A plastic gum-like composition was obtained and this composition set in about 7 minutes to produce a setting material having a high strength.

EXAMPLE 26 TO 37

2 grams of a powder indicated in Table 2 was mixed with 1 g of a setting solution indicated in Table 2 (Chitosan was "Flonac N" made by Kyowa Yushi Kogyo K.K.). Similar to the cases of Examples 21 to 25, plastic gum-like compositions were obtained and setting materials having a high strength could be produced from the compositions. The periods time required for these compositions to set are also shown in Table 2, in which $\alpha$-TCP and COP denote $\alpha$-tricalcium phosphate and tetracalcium phosphate, respectively.

TABLE 2

| Example | Powder composition | Composition of setting solution | | | | | | Time to set (min) |
|---|---|---|---|---|---|---|---|---|
| | | Aqueous acidic solution | | Polysaccharide | | Other additives | | |
| | | acid and its concentration | amount (g) | | amount (g) | | amount (g) | |
| 26 | α-TCP | 40% citric acid | 10 | chitosan | 0.1 | glycerin | 3 | 10 |
| 27 | COP | 40% citric acid | 10 | chitosan | 0.1 | sorbitol | 3 | 5 |
| 28 | 2:1 mixture of α-TCP + COP | 40% citric acid | 10 | chitosan | 0.1 | glucose | 3 | 7 |
| 29 | α-TCP | 40% malic acid | 10 | chitosan | 0.1 | — | — | 7 |
| 30 | α-TCP | 40% malic acid | 10 | chitosan | 0.1 | glucose | 3 | 10 |
| 31 | COP | 40% malic acid | 10 | chitosan | 0.1 | glycerin | 3 | 5 |
| 32 | 2:1 mixture of α-TCP + COP | 40% malic acid | 10 | chitosan | 0.1 | sorbitol | 3 | 7 |
| 33 | COP | 40% malic acid | 10 | chitosan | 0.1 | saccharose | 3 | 5 |
| 34 | α-TCP | 40% phosphoric acid | 10 | chitosan | 0.1 | saccharose +glycerin | 3+3 | 2 |
| 35 | COP | 25% polyacrylic acid | 10 | chitosan | 0.1 | — | — | 10 |
| 36 | α-TCP | 25% polyacrylic acid | 10 | chitosan | 0.1 | glycerin | 3 | 20 |
| 37 | 2:1 mixture of α-TCP + COP | 25% polyacrylic acid | 10 | chitosan | 0.1 | saccharose | 3 | 15 |

COMPARATIVE EXAMPLE 3

The same procedures as in Example 21 were repeated except that chitosan was not added to the setting solution. The composition was not gum-like and has a deteriorated formability although the composition hardened in about 2 minutes.

EXAMPLE 38

3 grams of the powder mixture of α-tricalcium phosphate and tetracalcium phosphate obtained in Example 17 was mixed with 2 g of a commercial aqueous solution of gluconic acid (made by Wako Pure Chemical Industries, Ltd.; concentration, ca. 50%). The mixture underwent a hydrolytic reaction and set in about 1 hour.

EXAMPLE 39

The powder prepared as in Example 38 was mixed with setting solutions (indicated in Table 3) at a weight ratio of 1/1 and setting materials were obtained. The setting times and the temperatures produced by the exothermic reaction of the setting process are also shown in Table 3.

TABLE 3

| Composition of setting solution (wt%) | | | | Setting time (min) | Temperature (°C) |
|---|---|---|---|---|---|
| Citric acid | Glycerin | Sorbitol | Saccharose | | |
| 45 | 10 | 0 | 0 | 0.75 | 55 |
| 45 | 20 | 0 | 0 | 2 | 51 |
| 45 | 30 | 0 | 0 | 8 | 45 |
| 45 | 0 | 10 | 0 | 0.8 | 53 |
| 45 | 0 | 20 | 0 | 2 | 51 |
| 45 | 0 | 0 | 10 | 1 | 52 |
| 45 | 0 | 0 | 20 | 2.8 | 49 |
| 45 | 0 | 0 | 0 | 0.5 | 66 |

EXAMPLE 40

The mixture of α-tricalcium phosphate and tetracalcium phosphate prepared in Example 38 was used as a powder component. 2 grams of this powder was mixed with 1 g of a setting solution prepared by dissolving 0.1 g of chitosan ("Flonac N" made by Kyowa Yushi Kogyo K.K.) in 10 g of an aqueous solution of 40% citric acid. A plastic gum-like composition was obtained and this composition set in about 2 minutes to produce a setting material having a high strength.

COMPARATIVE EXAMPLE 4

The same procedures as in Example 40 were repeated except that chitosan was not added to the setting solution. The composition obtained was not gum-like and had a deteriorated formability although the composition set in about 2 minutes.

EXAMPLE 41

The mixture of α-tricalcium phosphate and tetracalcium phosphate prepared in Example 38 was used as a powder component. 2 grams of this powder was mixed with 1 g of a setting solution prepared by dissolving 3 g of saccharose and 0.1 of chitosan ("FLonac N" made by Kyowa Yushi Kogyo K.K.) in 10 g of an aqueous solution of 40% citric acid. A plastic gum-like composition was obtained and this composition set in about 9 minutes to produce a setting material having a high strength.

COMPARATIVE EXAMPLE 5

The same procedures as in Example 41 were repeated except that chitosan was not added to the setting solution. The composition obtained was not gum-like and had a deteriorated formability although the composition set in about 2 minutes.

COMPARATIVE EXAMPLE 6

The same procedures as in Example 17 were repeated except that chitosan (Kimitsu Chitosan F made by Kimitsu Chemicals Co., Ltd.) was used instead of carboxymethyl chitin used in Example 17 so as to prepare a setting solution having a viscosity at room temperature of 50 cp. The composition obtained was not gum-like.

In accordance with one aspect of the present invention, a calcium phosphate compound is used as a powder component and mixed with a setting solution which is an aqueous acidic solution having a polysaccharide dissolved therein. The resulting composition is highly gum-like and can be readily formed into a desired shape before setting. The setting reaction which occurs is mild and it proceeds at a moderate rate and uniformly to produce a setting material having a high strength and which yet wll not harm body tissues.

A mixture of $\alpha$-tricalcium phosphate and tetracalcium phosphate can be readily prepared by calcining a calcium phosphate with a molar ratio of Ca/P of more than 1.5 and about 1.8 or less at a temperature of about from 1,150°C to 1,450 °C under reduced pressure. In this preferred embodiment, an aqueous acidic solution containing not only a polysaccharide but also at least one of glycerin, a monosaccharide, an oligosaccharide and a sugaralcohol may preferably be used as a setting solution. This offers further advantage of allowing the setting reaction to proceed more uniformly to a fuller extent at a moderate and mild rate, thereby producing a setting material having a high strength and which yet will not harm body tissues.

Therefore, the present invention provides a composition which is useful as a medical or dental cement material or a bone prosthetic material and which, immediately before use, is rendered sufficiently gum-like by mixing the powder and the setting solution to replace defects of a bone or tooth, and is subsequently set. In addition, the present invention provides a setting biomaterial of a desired shape such as a setting material in block form, an artificial bone or dental root.

## Claims

1. A composition for forming a calcium phosphate type setting material comprising
   (1) powder comprising at least one of $\alpha$-tricalcium phosphate and tetracalcium phosphate; and
   (2) a setting solution comprising an aqueous acidic solution having dissolved therein at least one polysaccharide selected from carboxymethyl chitin, glycol chitin, pullulan, high methoxy-pectin and chitosan in such an amount that said setting solution has a viscosity at room temperature of 70 mPa.s (70 cp) or more.

2. A composition as claimed in claim 1, wherein the acid concentration of said acidic aqueous solution is 25 wt% or more.

3. A composition as claimed in claim 2, wherein the acid concentration of said acidic aqueous solution is from 25 to 55 wt%.

4. A composition as claimed in claim 1, wherein said acidic aqueous solution is an aqueous solution of an organic acid.

5. A composition as claimed in claim 4, wherein said acidic aqueous solution is an aqueous solution of an organic acid selected from acetic acid, lactic acid, citric acid, malic acid, malonic acid, succinic acid, glutaric acid, tartaric acid, and polyacrylic acid.

6. A composition as claimed in claim 5, wherein said acidic aqueous solution is selected from a citric acid solution having an acid concentration of 40 wt%, a malic acid solution having an acid concentration of 40 wt%, and a polyacrylic acid solution having an acid concentration of 25 wt%.

7. A composition as claimed in claim 1, wherein said acidic aqueous solution is an aqueous solution of an inorganic acid.

8. A composition as claimed in claim 7, wherein said acidic aqueous solution is an aqueous solution of

14

phosphoric acid,

9. A composition as claimed in claim 8, wherein said acidic aqueous solution is a phosphoric acid solution having an acid concentration of 40 wt%.

10. A composition as claimed in claim 1, wherein said setting solution has a viscosity of from 100 to 20,000 mPa.s (100 to 20,000 cp.)

11. A composition as claimed in claim 10, wherein said setting solution has a viscosity of from 500 to 10,000 mPa.s (500 to 10,000 cp).

12. A composition as claimed in claim 1, wherein said polysaccharide is chitosan.

13. A composition as claimed in claim 1, wherein the ratio of said powder to said setting solution is from 0.4 to 2.7 by weight.

14. A composition as claimed in claim 13, wherein the ratio of said powder to said setting solution is from 0.4 to 2.0 by weight.

15. A composition as claimed in claim 1, wherein the ratio of the total amount of $\alpha$-tricalcium phosphate and tetracalcium phosphate to the total amount of the other components in said powder is 1/3 by weight or more.

16. A composition as claimed in claim 1, wherein said powder further comprises at least one of $\beta$-tricalcium phosphate and hydroxyapatite.

17. A composition as claimed in claim 1, wherein said powder is selected from $\alpha$-tricalcium phosphate, tetracalcium phosphate, a mixture of $\alpha$-tricalcium phosphate and tetracalcium phosphate, a mixture of $\alpha$-tricalcium phosphate and hydroxyapatite, a mixture of tetracalcium phosphate and hydroxyapatite, a mixture of tetracalcium phosphate and $\beta$-tricalcium phosphate, and a mixture of $\alpha$-tricalcium phosphate and $\beta$-tricalcium phosphate.

18. A composition as claimed in claim 17, wherein said powder is a mixture of $\alpha$-tricalcium phosphate and tetracalcium phosphate.

19. A composition as claimed in claim 18, wherein said powder is produced by the step of calcining, at a temperature of from about 1,150°C to 1,450°C under a pressure of 10 Pa or less, a calcium phosphate having a molar ratio of Ca/P of about 1.8 or less and more than 1.5.

20. A composition as claimed in claim 19, wherein said calcium phosphate has a molar ratio of Ca/P of from about 1.6 to 1.8.

21. A composition as claimed in claim 20, wherein said calcium phosphate is calcined under a pressure of $10^{-2}$ Pa or less.

22. A composition as claimed in claim 17, wherein said powder is a mixture of $\alpha$-tricalcium phosphate and hydroxyapatite.

23. A composition as claimed in claim 22, wherein said powder is produced by the step of calcining, at a temperature of 1,000°C or more, a calcium phosphate having a molar ratio of Ca/P of less than 1.67 and more than 1.5.

24. A composition as claimed in claim 23, wherein said powder is produced by the step of calcining, at a temperature of from 1,150 to 1,450°C, a calcium phosphate having a molar ratio of Ca/p of from 1.62 to 1.54.

25. A composition as claimed in claim 1, wherein said setting solution further comprises at least one of a monosaccharide, an oligosaccharide, a sugaralcohol and a polyhydric alcohol.

15

EP 0 323 632 B1

**26.** A composition as claimed in claim 25, wherein the concentration of said at least one of a monosaccharide, an oligosaccharide, a sugaralcohol and a polyhydric alcohol in said setting solution is from 5 to 40 wt%.

**27.** A composition as claimed in claim 26, wherein the concentration of said at least one of a monosaccharide, an oligosaccharide, a sugaralcohol and a polyhydric alcohol in said setting solution is from 10 to 30 wt%.

**28.** A process for producing a calcium phosphate type setting material comprising the step of mixing
(1) powder comprising at least one of α-tricalcium phosphate and tetracalcium phosphate; and
(2) a setting solution comprising an aqueous acidic solution having dissolved therein at least one polysaccharide selected from carboxymethyl chitin, glycol chitin, pullulan, high methoxy-pectin and chitosan in such an amount that said setting solution has a viscosity of 70 mPa.s (70 cp) or more, to form a setting material.

**Revendications**

**1.** Composition pour former une matière durcissable du type phosphate de calcium comprenant :
(1) une poudre comportant au moins un des deux composés : phosphate tricalcique α et phosphate tétracalcique; et
(2) une solution durcissable comprenant une solution aqueuse acide dans laquelle est en solution au moins un polysaccharide choisi parmi la carboxyméthyl chitine, la glycol chitine, le pullulane, une pectine à teneur élevée en méthoxy et du chitosane, en quantité telle que cette solution durcissable a une viscosité de 70 mPa.s (70 cp) ou davantage, à la température ambiante.

**2.** Composition selon la revendication 1, dans laquelle la concentration en acide de ladite solution aqueuse acide est de 25 % en poids ou davantage.

**3.** Composition selon la revendication 2, dans laquelle la concentration en acide de ladite solution aqueuse acide est de 25 à 55 % en poids.

**4.** Composition selon la revendication 1, dans laquelle ladite solution aqueuse acide est une solution aqueuse d'un acide organique.

**5.** Composition selon la revendication 4, dans laquelle ladite solution aqueuse acide est une solution aqueuse d'un acide organique choisi parmi l'acide acétique, l'acide lactique, l'acide citrique, l'acide malique, l'acide malonique, l'acide succinique, l'acide glutarique, l'acide tartrique, et l'acide polyacrylique.

**6.** Composition selon la revendication 5, dans laquelle ladite solution aqueuse acide est choisie parmi les suivantes : solution d'acide citrique ayant une concentration en acide de 40 % en poids, solution d'acide malique ayant une concentration en acide de 40 % en poids, et solution d'acide polyacrylique ayant une concentration en acide de 25 % en poids.

**7.** Composition selon la revendication 1, dans laquelle ladite solution aqueuse acide est une solution aqueuse d'un acide minéral.

**8.** Composition selon la revendication 7, dans laquelle ladite solution aqueuse acide est une solution aqueuse d'acide phosphorique.

**9.** Composition selon la revendication 8, dans laquelle ladite solution aqueuse acide est une solution d'acide phosphorique ayant une concentration en acide de 40 % en poids.

**10.** Composition selon la revendication 1, dans laquelle ladite solution durcissable a une viscosité de 100 à 20 000 mPa.s (100 à 20 000 cp.).

**11.** Composition selon la revendication 10, dans laquelle ladite solution durcissable a une viscosité de 500 à 10 000 mPa.s (500 à 10 000 cp.).

16

**12.** Composition selon la revendication 1, dans laquelle ledit polysaccharide est le chitosane.

**13.** Composition selon la revendication 1, dans laquelle la proportion de ladite poudre par rapport à ladite solution durcissable est de 0,4 à 2,7 % en poids.

**14.** Composition selon la revendication 13, dans laquelle la proportion de ladite poudre par rapport à ladite solution durcissable est de 0,4 à 2,0 % en poids.

**15.** Composition selon la revendication 1, dans laquelle la proportion de la quantité totale de phosphate tricalcique $\alpha$ et de phosphate tétracalcique par rapport à la quantité totale des autres constituants de ladite poudre est de 1/3 en poids ou davantage.

**16.** Composition selon la revendication 1, dans laquelle ladite poudre contient en outre au moins l'un des deux composés phosphate tricalcique $\beta$ et hydroxyapatite.

**17.** Composition selon la revendication 1, dans laquelle ladite poudre est choisie parmi le phosphate tricalcique $\alpha$, le phosphate tétracalcique, un mélange de phosphate tricalcique $\alpha$ et de phosphate tétracalcique, un mélange de phosphate tricalcique $\alpha$ et d'hydroxyapatite, un mélange de phosphate tétracalcique et d'hydroxyapatite, un mélange de phosphate tétracalcique et de phosphate tricalcique $\beta$ et un mélange de phosphate tricalcique $\alpha$ et de phosphate tricalcique $\beta$.

**18.** Composition selon la revendication 17, dans laquelle ladite poudre est un mélange de phosphate tricalcique $\alpha$ et de phosphate tétracalcique.

**19.** Composition selon la revendication 18, dans laquelle ladite poudre est produite par l'opération de calcination à une température d'environ 1150°C à 1450°C, sous une pression de 10 Pa ou moins, d'un phosphate de calcium ayant un rapport molaire Ca/P d'environ 1,8, ou moins, et supérieur à 1,5.

**20.** Composition selon la revendication 19, dans laquelle ledit phosphate de calcium a un rapport molaire Ca/P d'environ 1,6 à 1,8.

**21.** Composition selon la revendication 20, dans laquelle ledit phosphate de calcium est calciné sous une pression de $10^{-2}$ Pa ou moins.

**22.** Composition selon la revendication 17, dans laquelle ladite poudre est un mélange de phosphate tricalcique $\alpha$ et d'hydroxyapatite.

**23.** Composition selon la revendication 22, dans laquelle ladite poudre est produite par l'opération de calcination, à une température de 1000°C ou davantage, d'un phosphate de calcium ayant un rapport molaire Ca/P inférieur à 1,67 et supérieur à 1,5.

**24.** Composition selon la revendication 23, dans laquelle ladite poudre est produite par l'opération de calcination, à une température de 1150 à 1450°C, d'un phosphate de calcium ayant un rapport molaire Ca/P de 1,62 à 1,54.

**25.** Composition selon la revendication 1, dans laquelle ladite solution durcissable comprend en outre au moins l'un des composés suivants : monosaccharide, oligosaccharide, sucre alcool et alcool polyhydrique.

**26.** Composition selon la revendication 25, dans laquelle la concentration dudit composé choisi parmi les suivants : monosaccharide, oligosaccharide, sucre alcool et alcool polyhydrique, dans cette solution durcissable est de 5 à 40 % en poids.

**27.** Composition selon la revendication 6, dans laquelle la concentration dudit composé choisi parmi les suivants : oligosaccharide, sucre alcool et alcool polyhydrique, dans cette solution durcissable est de 10 à 30 % en poids.

**28.** Procédé de préparation d'une matière durcissable du type phosphate de calcium, comprenant l'opéra-

17

EP 0 323 632 B1

tion consistant à mélanger :

(1) une poudre comprenant au moins un des deux composés : phosphate tricalcique α et phosphate tétracalcique; et

(2) une solution durcissable comportant une solution acide aqueuse dans laquelle est dissoute au moins un polysaccharide choisi parmi la carboxyméthyl chitine, la glycol chitine, le pullulane, une pectine à teneur élevée en méthoxy et un chitosane, en quantité telle que cette solution durcissable a une viscosité de 70 mPa.s (70 cp) ou davantage, pour former une matière durcissable.

**Patentansprüche**

1.  Zusammensetzung zur Bildung eines kalziumphosphatartigen Abbindematerials, umfassend:

    (1) Pulver, umfassend alpha-Trikalziumphosphat und/oder Tetrakalziumphosphat und

    (2) eine Abbindelösung, umfassend eine wäßrige Lösung, in welcher wenigstens ein Polysaccharid, ausgewählt aus Carboxymethylchitin, Glykolchitin, Pullulan, Hochmethoxy-Pektin und Chitosin in einer solchen Menge gelöst ist, daß die Abbindelösung eine Viskosität bei Raumtemperatur von 70 mPa.s (70 cp) oder mehr hat.

2.  Zusammensetzung gemäß Anspruch 1, in welcher die Säurekonzentration der sauren wäßrigen Lösung 25 Gew.-% oder mehr beträgt.

3.  Zusammensetzung gemäß Anspruch 2, in welcher die Säurekonzentration der sauren wäßrigen Lösung von 25 bis 55 Gew.-% beträgt.

4.  Zusammensetzung gemäß Anspruch 1, in welcher die saure wäßrige Lösung eine wäßrige Lösung einer organischen Säure ist.

5.  Zusammensetzung gemäß Anspruch 4, in welcher die saure wäßrige Lösung eine wäßrige Lösung einer organischen Säure, ausgewählt aus Essigsäure, Milchsäure, Zitronensäure, Apfelsäure, Malonsäure, Bernsteinsäure, Glutarsäure, Weinsäure und Polyacrylsäure ist.

6.  Zusammensetzung gemäß Anspruch 5, in welcher die saure wäßrige Lösung ausgewählt ist aus einer Zitronensäurelösung mit einer Säurekonzentration von 40 Gew.-%, einer Apfelsäurelösung mit einer Säurekonzentration von 40 Gew.-% und einer Polyacrylsäurelösung mit einer Säurekonzentration von 25 Gew.-%.

7.  Zusammensetzung gemäß Anspruch 1, in welcher die saure wäßrige Lösung eine wäßrige Lösung einer anorganischen Säure ist.

8.  Zusammensetzung gemäß Anspruch 7, in welcher die saure wäßrige Lösung eine wäßrige Lösung von Phosphorsäure ist.

9.  Zusammensetzung gemäß Anspruch 8, in welcher die saure wäßrige Lösung eine Phosphorsäurelösung mit einer Säurekonzentration von 40 Gew.-% ist.

10. Zusammensetzung gemäß Anspruch 1, in welcher die Abbindelösung eine Viskosität von 100 bis 20.000 mPa.s (100 bis 20.000 cp) hat.

11. Zusammensetzung gemäß Anspruch 10, in welcher die Abbindelösung eine Viskosität von 500 bis 10.000 mPa.s (500 bis 10.000 cp) hat.

12. Zusammensetzung gemäß Anspruch 1, in welcher das Polysaccharid Chitosan ist.

13. Zusammensetzung gemäß Anspruch 1, in welcher das Gewichtsverhältnis des Pulvers zu der Abbindelösung 0,4 bis 2,7 beträgt.

14. Zusammensetzung gemäß Anspruch 13, in welcher das Gewichtsverhältnis des Pulvers zu der Abbindelösung 0,4 bis 2,0 beträgt.

18

**15.** Zusammensetzung gemäß Anspruch 1, in welcher das Gewichtsverhältnis der Gesamtmenge von alpha-Trikalziumphosphat und Tetrakalziumphosphat zu der Gesamtmenge der anderen Komponenten in dem Pulver 1/3 oder mehr beträgt.

**16.** Zusammensetzung gemäß Anspruch 1, in welcher das Pulver weiterhin wenigstens beta-Trikalziumphosphat und/oder Hydroxylapatit umfaßt.

**17.** Zusammensetzung gemäß Anspruch 1, in welcher das Pulver ausgewählt ist aus alpha-Trikalziumphosphat, Tetrakalziumphosphat, eine Mischung aus alpha-Trikalziumphosphat und Tetrakalziumphosphat, eine Mischung aus alpha-Trikalziumphosphat und Hydroxylapatit, einer Mischung aus Tetrakalziumphosphat und Hydroxylapatit, einer Mischung aus Tetrakalziumphosphat und beta-Trikalziumphosphat und einer Mischung aus alpha-Trikalziumphosphat und beta-Trikalziumphosphat.

**18.** Zusammensetzung gemäß Anspruch 17, in welcher das Pulver eine Mischung aus alpha-Trikalziumphosphat und Tetrakalziumphosphat ist.

**19.** Zusammensetzung gemäß Anspruch 18, in welcher das Pulver hergestellt wurde durch die Stufe des Kalzinierens bei einer Temperatur von etwa 1.150°C bis 1.450°C unter einem Druck von 10 Pa oder weniger eines Kalziumphosphats mit einem molaren Verhältnis von Ca/P von etwa 1,8 oder weniger und mehr als 1,5.

**20.** Zusammensetzung gemäß Anspruch 19, in welcher das Kalziumphosphat ein molares Verhältnis von Ca/P von etwa 1,6 bis 1,8 hat.

**21.** Zusammensetzung gemäß Anspruch 20, in welcher das Kalziumphosphat unter einem Druck von $10^{-2}$ Pa oder weniger kalziniert wurde.

**22.** Zusammensetzung gemäß Anspruch 17, in welcher das Pulver eine Mischung aus alpha-Trikalziumphosphat und Hydroxylapatit ist.

**23.** Zusammensetzung gemäß Anspruch 22, in welcher das Pulver hergestellt wurde durch die Stufe des Kalzinierens bei einer Temperatur von 1.000°C oder mehr eines Kalziumphosphats mit einem molaren Verhältnis von Ca/P von weniger als 1,67 und mehr als 1,5.

**24.** Zusammensetzung gemäß Anspruch 23, in welcher das Pulver hergestellt wurde durch die Stufe des Kalzinierens bei einer Temperatur von 1.150 bis 1.450°C eines Kalziumphosphats mit einem molaren Verhältnis von Ca/P von 1,62 bis 1,54.

**25.** Zusammensetzung gemäß Anspruch 1, in welcher die Abbindelösung weiterhin wenigstens eine Verbindung, ausgewählt aus einem Monosaccharid, einem Oligosaccharid, einem Zuckeralkohol und einem mehrwertigen Alkohol umfaßt.

**26.** Zusammensetzung gemäß Anspruch 25, in welcher die Konzentration der wenigstens einen Verbindung, ausgewählt aus einem Monosaccharid, einem Oligosaccharid, einem Zuckeralkohol und einem mehrwertigen Alkohol in der Abbindelösung 5 bis 40 Gew.-% beträgt.

**27.** Zusammensetzung gemäß Anspruch 26, in welcher die Konzentration der wenigstens einen Verbindung, ausgewählt aus einem Monosaccharid, einem Oligosaccharid, einem Zuckeralkohol und einem mehrwertigen Alkohol in der Abbindelösung 10 bis 30 Gew.-% beträgt.

**28.** Verfahren zur Herstellung eines kalziumphosphatartigen Abbindematerials, umfassend die Stufen des Vermischens von
    (1) ein Pulver, umfassend wenigstens alpha-Trikalziumphosphat und/oder Tetrakalziumphosphat und
    (2) einer Abbindelösung, umfassend eine wäßrige saure Lösung, in welcher wenigstens ein Polysaccharid, ausgewählt aus Carboxymethylchitin, Glykolchitin, Pullulan, Hochmethoxy-Pektin und Chitosan in einer solchen Menge gelöst ist, daß die Abbindelösung eine Viskosität von 70 mPa.s (70 cp) oder mehr hat, unter Ausbildung eines Abbindematerials.

COUNTS

Fig. 2

2θ[°]

Fig. 3

EP 0 323 632 B1